# EUROPEAN PATENT SPECIFICATION

(11) **EP 3 375 770 B1**
(45) Date of publication and mention of the grant of the patent: **03.06.2020**
(21) Application number: 16864047.2
(22) Date of filing: 28.10.2016
(51) Int. Cl.: C07C 31/133, A61K 8/34, A61Q 5/00, A61Q 5/02, A61Q 13/00, C07C 29/32, C11B 9/00, C11D 3/20, C11D 3/50, D06M 13/144

(54) **CYCLOPROPANE COMPOUND**
CYCLOPROPANVERBINDUNG
COMPOSÉ CYCLOPROPANE

(30) Priority: 11.11.2015 JP 2015221537
(43) Date of publication of application: 19.09.2018
(73) Proprietor: Kao Corporation, Chuo-ku Tokyo 103-8210 (JP)
(72) Inventor: UEDA, Junko, Wakayama-shi Wakayama 640-8580 (JP); TOKI, Naotoshi, Sumida-ku Tokyo 131-8501 (JP); ASADA, Takahiro, Wakayama-shi Wakayama 640-8580 (JP); SATO, Makiko, Sumida-ku Tokyo 131-8501 (JP)
(74) Representative: Hoffmann Eitle
(86) International application number: PCT/JP2016/082084
(87) International publication number: WO 2017/082084

(56) References cited:
- WO-A1-2009/023980
- JP-A- 2007 106 721
- JP-A- 2008 525 324
- JP-A- 2010 536 802
- JP-A- 2014 522 395
- KIYOTA, HIROMASA et al.: "Syntheses and odour descriptions of jasmine lactone and cis-jasmone analogues bearing cyclopropane moieties", FLAVOUR AND FRAGRANCE JOURNAL, vol. 16, no. 3, 1 January 2001 (2001-01-01), pages 175-179, XP055541908, DOI: 10.1002/ffj.974
- HIROMASA KIYOTA et al.: "Dai 45 Kai Symposium on the Chemistry of Terpenes", Essential oils and Aromatics Koen Yoshishu, 1 October 2001 (2001-10-01), pages 111-113, XP009511185,
- DATABASE CAS 17 June 2016 (2016-06-17), retrieved from STN Database accession no. 1933785-25-1
- DATABASE CAS 5 February 2016 (2016-02-05), retrieved from STN Database accession no. 1860506-28-0

## Description

### CYCLOPROPANE COMPOUND

### [Technical Field]

The present invention relates to a new cyclopropane compound and a fragrance composition containing said cyclopropane compound.

### [Background Art]

Fragrance is an important element that creates, for example, preference, a sense of luxury, a sense of ease, and expectations for the effect, with respect to products and the like. Furthermore, a distinctive fragrance provides an effect of identifying products and an effect of attracting customers. Particularly, floral fragrance notes characterized by softness are used for various products including toiletry products.

On the other hand, in order to control, for example, a long-lasting property and balance of fragrance, generally, a fragrance is imparted to a product using a fragrance composition in which a plurality of fragrance ingredients are mixed together. It is required for the fragrance ingredients composing the fragrance composition to be highly harmonious with other fragrance ingredients. Particularly, the floral fragrance note is known to have excellent harmony with other fragrance ingredients due to the soft fragrance thereof.

Patent Document 1 discloses that, for example, (cis, trans)-1-(2-ethyl-1-methylcyclopropyl)hexan-1-ol has green, fresh, spice-like, and chocolate odors. It discloses ((cis)-2-isobutyl-1-methylcyclopropyl)methanol but does not describe the odor thereof.

Patent Document 2 discloses that, for example, (1R*, 2R*)-[2-methyl-2-(4'-methyl-3'-pentenyl)cyclopropenyl]methanol has a heavy rose-like odor with a slightly woody dimension.

Patent Document 3 discloses, for example, 4-(2,2-dimethyl-cyclopropyl)-butan-2-ol but does not describe the odor thereof.

Fragrance ingredients tend to have similar fragrance notes when they have similar structures to each other, but there are many exceptions. Particularly, when a plurality of substituents are combined to change the fragrance note, it is difficult to predict how the fragrance note will change and it also is difficult to predict the harmony with other fragrance ingredients.

### [Prior Art Documents]

### [Patent Documents]

[Patent Document 1] JP2010-536802A
[Patent Document 2] JP2007-106721A
[Patent Document 3] JP2008-525324A

### [SUMMARY OF THE INVENTION]

### [Problem to be Solved by the Invention]

Some fragrance ingredients may interfere with other fragrance components and thereby the fragrance notes thereof may change or weaken one another. Therefore, in order to increase the degree of freedom in blending odors, it is desirable to expand variations of fragrance ingredients. Particularly, there have been demands for a fragrance composition that has a floral fragrance note with softness and that is suitably used for imparting fragrance to various products including toiletry products.

The present invention is intended to provide a compound and a fragrance composition that are excellent in harmony with various other fragrance materials and can be blended to provide a natural and fresh floral feeling.

### [Means for Solving Problem]

The present inventors found that a cyclopropane compound with a particular structure has a floral fragrance note, is excellent in harmony with various other fragrance materials, and can be blended to provide a natural and fresh floral feeling. Thus, the present invention was completed.

That is, the present invention is a new cyclopropane compound represented by Formula (I) and a fragrance composition containing said cyclopropane compound.

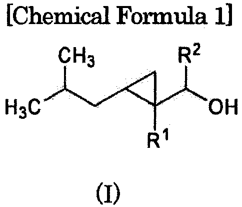

In Formula (I) above, R¹ is a methyl group and R² is a methyl group, or R¹ is a hydrogen atom and R² is an ethyl group.

### [Effects of the Invention]

The cyclopropane compound of the present invention has a floral fragrance note, is excellent in harmony with various other fragrance materials, and can be blended to provide a natural and fresh floral feeling.

### [Description of Preferred Embodiments]

### [Cyclopropane Compound]

The cyclopropane compound of the present invention is one represented by Formula (I) below. In the present specification, the cyclopropane compound of Formula (I) may be referred to as "Compound (I)."

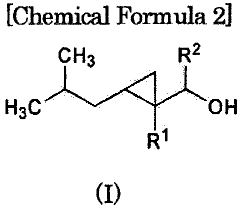

[In Formula (I), R¹ is a methyl group and R² is a methyl group, or R¹ is a hydrogen atom and R² is an ethyl group.]

Examples of the cyclopropane compound of the present invention include a compound represented by Formula (I) where R¹ is a methyl group and R² is a methyl group, and a compound represented by Formula (I) where R¹ is a hydrogen atom and R² is an ethyl group. From the viewpoints of being excellent in harmony with various other fragrance materials and being blended to provide a fresh and juicy floral feeling, the cyclopropane compound of the present invention is preferably 1-(2-isobutyl-1-methylcyclopropyl)ethan-1-ol (represented by Formula (I-1) below and may be referred to as Compound (I-1) in the present specification) represented by Formula (I) where R¹ is a methyl group and R² is a methyl group among the 1-(2-isobutyl-1-methylcyclopropyl)ethan-1-ol (I-1) represented by Formula (I) where R¹ is a methyl group and R² is a methyl group and 1-(2-isobutylcyclopropyl)propan-1-ol (represented by Formula (I-2) below and may be referred to as Compound (I-2) in the present specification) represented by Formula (I) where R¹ is a hydrogen atom and R² is an ethyl group.

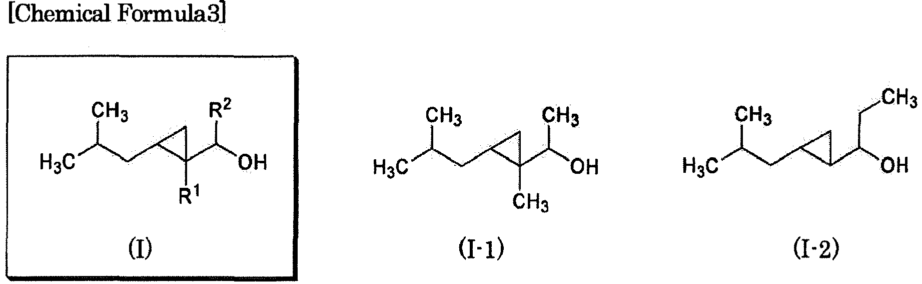

### [Method of Producing Cyclopropane Compound]

The cyclopropane compound represented by Formula (I) of the present invention can be synthesized using a general organic chemical reaction, and the method of producing it is not limited. For example, the double bond moiety of the compound represented by Formula (II) can be cyclopropanated to obtain a compound represented by Formula (I) (see Scheme 1).

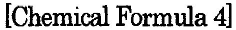

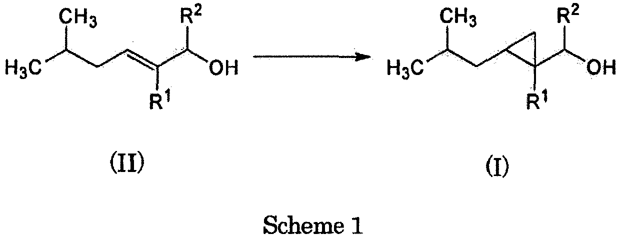

In Formula (I) above, R¹ is a methyl group and R² is a methyl group, or R¹ is a hydrogen atom and R² is an ethyl group.

The method of cyclopropanating the double bond of the compound represented by Formula (II) is not particularly limited as long as it is a general method. Examples thereof to achieve it include a method of reacting diiodomethane in a solvent in the presence of a metal catalyst such as a zinc catalyst or under ultrasonic irradiation, or a method of reacting diazomethane in a solvent in the presence of lead acetate. Particularly, the method of cyclopropanation is preferably the method of reacting diiodomethane in a solvent in the presence of a metal catalyst or under ultrasonic irradiation, more preferably the method of reacting diiodomethane in the presence of a metal catalyst.

The metal catalyst used in the method of reacting diiodomethane in a solvent in the presence of a metal catalyst or under ultrasonic irradiation is preferably a zinc catalyst, more preferably dialkylzinc such as dimethylzinc or diethylzinc.

The amount of the metal catalyst to be used in the method of reacting diiodomethane in a solvent in the presence of a metal catalyst or under ultrasonic irradiation is preferably 0.01 times by mol or more, more preferably 0.1 times by mol or more, but is preferably 10 times by mol or less, more preferably 5 times by mol or less, with respect to the compound (II).

Examples of the solvent used in the method of reacting diiodomethane in a solvent in the presence of a metal catalyst or under ultrasonic irradiation include dichloromethane and diethyl ether.

The compound represented by Formula (I) obtained after completion of the reaction may be subjected to washing with water, evaporating the solvent to dryness, purification by column chromatography, etc. These operations can be performed to obtain a compound represented by Formula (I) of high purity.

### [Production of Compound of Formula (II)]

The compound of Formula (II) can be obtained as follows: for example, isovaleraldehyde (represented by Formula (XI) below and may be referred to as Compound (XI) in the present specification) and 2-butanone (represented by Formula (X) below and may be referred to as Compound (X) in the present specification) are used to perform a cross-aldol addition reaction, followed by dehydration to obtain a compound of Formula (III), and then the compound of Formula (III) is reduced (see Scheme 2). The compound of Formula (III) contains a compound of Formula (III-1) and a compound of Formula (III-2), and the compound of Formula (II) contains a compound of Formula (II-1) and a compound of Formula (II-2).

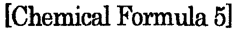

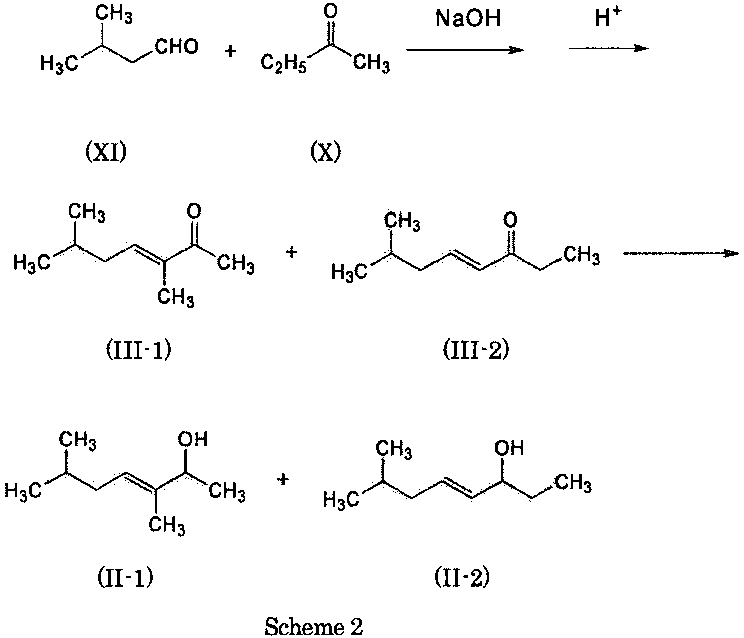

### <Cross-Aldol Addition Reaction and Dehydration Reaction>

In the above-mentioned production method, isovaleraldehyde (XI) and 2-butanone (X) were first used to perform a cross-aldol addition reaction in the presence of a base catalyst. Preferably, the reaction is performed in an aqueous solution. The base catalyst is preferably an alkali metal hydroxide, more preferably sodium hydroxide. The reaction temperature is preferably 5°C to 30°C and the reaction time is preferably one hour to 60 hours.

Water may be further added to the reaction solution from the viewpoint of advancing the cross-aldol addition reaction with good yield.

The resultant product is then dehydrated. Preferably, the dehydration reaction is performed at or above the boiling point of water, with, for example, phosphoric acid being added to the reaction solution. When the pressure of the reaction system is changed, it is preferable to perform the reaction at or above the boiling point of water at said pressure. At 1 atm (101 kPa), it is preferable to perform the reaction between 105°C and 160°C.

The dehydration results in 3,6-dimethylhept-3-en-2-one (represented by Formula (III-1) above and may be referred to as Compound (III-1) in the present specification) and 7-methyloct-4-en-3-one (represented by Formula (III-2) below and may be referred to as Compound (III-2) in the present specification), which are α, β unsaturated ketones.

In the cross-aldol addition reaction and dehydration reaction, Compound (III-1) and Compound (III-2) are obtained (see Scheme 3). However, Compound (III-1) and Compound (III-2) can also be separated from each other by precision distillation or a combination of a silica gel column and distillation. This separation can result in highly pure Compound (III-1) and Compound (III-2). Alternatively, the separation conditions can be adjusted to obtain a mixture of Compound (III-1) and Compound (III-2) at a specific ratio.

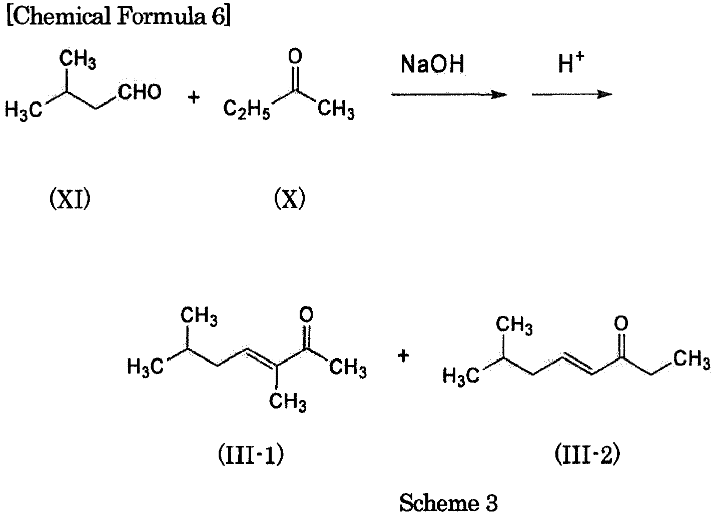

### <Reduction Reaction>

Next, the resultant a, B unsaturated ketones are reduced. The reduction method is not particularly limited as long as it is a general reduction method, but hydride reduction is preferable in order to reduce only the carbonyl group.

In the hydride reduction, it is preferable to use metal hydride such as sodium borohydride.

Furthermore, in the hydride reduction, it is preferable to further use a cerium compound in addition to the metal hydride in order to increase the selectivity of the reduction. The cerium compound is preferably cerium chloride.

Preferably, the hydride reduction is performed under a nitrogen atmosphere.

The reaction temperature for the hydride reduction is preferably -20°C to 50°C from the viewpoint of suppressing side reactions.

Preferably, the hydride reduction is performed in a solvent. The solvent is preferably a protic solvent such as water or alcohol.

By the reduction reaction, 3,6-dimethylhept-3-en-2-ol (represented by Formula (II-1) above and may be referred to as Compound (II-1) in the present specification) is obtained from Compound (III-1), while 7-methyloct-4-en-3-ol (represented by Formula (II-2) below and may be referred to as Compound (II-2) in the present specification is obtained from Compound (III-2) (see Scheme 4).

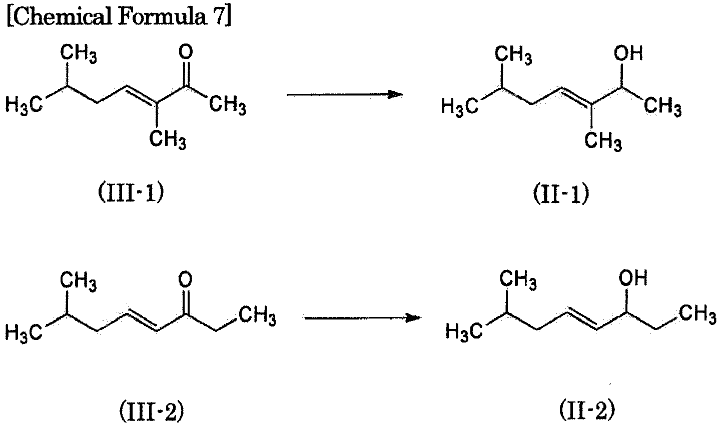

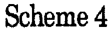

When Compound (III-1) and Compound (III-2) are not separated from each other and a mixture thereof is used to perform reduction, a mixture of Compound (II-1) and Compound (II-2) can be obtained.

Resultant Compound (I-1) has fragrance notes of floral, lilac, rose, and citrus. Compound (I-2) has fragrance notes of floral, herbal, fruity, and slightly muguet.

When Compound (II-1) and Compound (II-2) are not separated from each other and a mixture thereof is used to perform cyclopropanation, a mixture of Compound (I-1) and Compound (I-2) can be obtained.

### [Fragrance Composition]

The fragrance composition of the present invention includes Compound (I). The content of Compound (I) in the fragrance composition is preferably 0.01% by mass or more, more preferably 0.1% by mass or more, and further preferably 0.3% by mass or more, but preferably 99.99% by mass or less, more preferably 15% by mass or less, and further preferably 5% by mass or less. When Compound (I) is contained in an amount of 0.01% by mass to 99.99% by mass, a natural and fresh floral feeling can be imparted to the fragrance composition. Compound (I) is preferably Compound (I-1) from the viewpoint of being excellent in harmony with various other fragrance materials and of being able to be blended to impart a fresh and juicy floral feeling.

In addition, the fragrance composition of the present invention may contain both Compound (I-1) and Compound (I-2) as Compound (I). When the fragrance composition of the present invention contains Compound (I-1) and Compound (I-2), the total content of Compound (I-1) and Compound (I-2) in the fragrance composition is preferably 0.01% by mass or more, more preferably 0.1% by mass or more, and further preferably 0.3% by mass or more, but preferably 99.99% by mass or less, more preferably 15% by mass or less, and further preferably 5% by mass or less. When the fragrance composition contains Compound (I-1) and Compound (I-2) in a total amount of 0.01% by mass to 99.99% by mass, the fragrance composition can be provided with a floral feeling with fruity sweetness in addition to the natural and fresh juicyiness.

Furthermore, the fragrance composition of the present invention can contain, as fragrance materials other than Compound (I), other fragrance components commonly used or a blended fragrance material with a desired composition. When fragrance materials other than Compound (I) are contained, various floral odors such as a bouquet tone, hyacinth tone, geranium tone, rose tone, orchid tone, and lily of the valley tone (muguet) can be imparted to the fragrance composition of the present invention.

When the fragrance composition of the present invention contains, as Compound (I), both Compound (I-1) and Compound (I-2), the content ratio of Compound (I-1) to Compound (I-2) (Compound (I-1)/Compound (I-2)) (mass ratio) is preferably 70/30 or more, more preferably 80/20 or more, and further preferably 90/10 or more, from the viewpoint of strengthening the fresh and juicy floral feeling, but is preferably 99.9/0.1 or less, more preferably 99/1 or less, further preferably 98/2 or less, and still further preferably 95/5 or less, from the viewpoint of imparting fruity sweetness while maintaining the fresh and juicy floral fragrance note.

Furthermore, when both Compound (I-1) and Compound (I-2) are contained in a fiber treatment composition such as a softener, a cleaner composition, or a cosmetic composition such as a hair cosmetic of the present invention, the content ratio (mass ratio) of Compound (I-1) to Compound (I-2) is more preferably 90/10 to 95/5 from the viewpoint of imparting a natural and fresh floral fragrance note.

In the fragrance composition of the present invention, examples of other fragrance materials that can be used in combination with Compound (I) include fragrance components such as alcohols, hydrocarbons, phenols, esters, carbonates, aldehydes, ketones, acetals, ethers, carboxylic acids, lactones, nitriles, Schiff bases, natural essential oils, and natural extracts other than Compound (I).

Among these, as other fragrance materials other than Compound (I), alcohols, hydrocarbons, phenols, esters, aldehydes, ketones, acetals, and ethers are preferable, and particularly, alcohols and esters other than Compound (I) are more preferable,.

Examples of the alcohols other than Compound (I) include terpene-based alcohols, aromatic alcohols, and aliphatic alcohols.

Examples of the terpene-based alcohols include linalool, ethyllinalool, citronellol, geraniol, nerol, terpineol, α-terpineol, dihydromyrcenol, farnesol, nerolidol, cedrol, menthol, borneol, and isobornylcyclohexanol.

Examples of the aromatic alcohols include phenylethyl alcohol, benzyl alcohol, dimethyl benzyl carbinol, phenylethyl dimethyl carbinol, and phenyl hexanol.

### Examples of the aliphatic alcohols include cis-3-hexenol,

1-(2,2,6-trimethylcyclohexyl)-3-hexanol, AMBER CORE (Trade Name of Kao Corporation, 1-(2-tert-butyl cyclohexyloxy)-2-butanol), SANDALMYSORE CORE (Trade Name of Kao Corporation, 2-methyl-4-(2,2,3-trimethyl-3-cyclopneten-1-yl)-2-buten-1-ol), MAGNOL (Trade Name of Kao Corporation, a mixture containing ethylnorbornylcyclohexanol as a main component), and UNDECAVERTOL (Trade Name of Givaudan, 4-methyl-3-decene-5-ol).

Examples of the hydrocarbons include limonene, α-pinene, β-pinene, terpinene, cedrene, longifolene, and valencene.

Examples of the phenols include guaiacol, eugenol, isoeugenol, thymol, p-cresol, vanillin, and ethyl vanillin.

Examples of the esters include formate ester, acetate ester, propionate ester, butyrate ester, valerate ester, hexanoate ester, heptanoate ester, nonenoate ester, benzoate ester, cinnamate ester, salicylate ester, brassylate ester, tiglate ester, jasmonate ester, dihydrojasmonate ester, glycidate ester, and anthranilate ester.

Among these esters, acetate ester, propionate ester, and dihydrojasmonate ester are used preferably.

Examples of the formate ester include linalyl formate, citronellyl formate, and geranyl formate.

Examples of the acetate ester include hexyl acetate, cis-3-hexenyl acetate, linalyl acetate, citronellyl acetate, geranyl acetate, neryl acetate, terpinyl acetate, nopyl acetate, bornyl acetate, isobornyl acetate, acetyl eugenol, acetyl isoeugenol, o-tert-butylcyclohexyl acetate, p-tert-butylcyclohexyl acetate, tricyclodecenyl acetate, benzyl acetate, phenylethyl acetate, styralyl acetate, cinnamyl acetate, dimethylbenzylcarbinyl acetate, and 3pentyltetrahydropyran-4-yl acetate.

Examples of the propionate ester include citronellyl propionate, tricyclodecenyl propionate, allylcyclohexyl propionate, benzyl propionate, and styralyl propionate.

Examples of the butyrate ester include citronellyl butyrate, dimethylbenzylcarbinyl n-butyrate, and tricyclodecenyl isobutyrate.

Examples of the valerate ester include methyl valerate, ethyl valerate, butyl valerate, amyl valerate, benzyl valerate, and phenylethyl valerate. Examples of the hexanoate ester include methyl hexanoate, ethyl hexanoate, allyl hexanoate, linalyl hexanoate, and citronellyl hexanoate.

Examples of the heptanoate ester include methyl heptanoate and allyl heptanoate.

Examples of the nonenoate ester include methyl 2-nonenoate, ethyl 2-nonenoate, and ethyl 3-nonenoate.
Examples of the phenylacetate ester include phenylethyl phenyl acetate and p-cresyl phenylacetate.

Examples of the benzoate ester include methyl benzoate and benzyl benzoate.

Examples of the cinnamate ester include methyl cinnamate and benzyl cinnamate.

Examples of the salicylate ester include methyl salicylate, n-hexyl salicylate, cis-3-hexenyl salicylate, cyclohexyl salicylate, and benzyl salicylate.

Examples of the brassylate ester include ethylene brassylate.

Examples of the tiglate ester include geranyl tiglate, 1-hexyl tiglate, and cis-3-hexenyl tiglate.

Examples of the jasmonate ester include methyl jasmonate.

Examples of the dihydrojasmonate ester include methyl dihydrojasmonate.

Examples of the anthranilate ester include methyl anthranilate, ethyl anthranilate, and dimethyl anthranilate (methyl N-methyl anthranilate).

Examples of other esters include ethyl 2-methyl butyrate, methyl atrarate, allyl cyclohexyl glycolate, ALLYLAMYL GLYCOLATE (Trade Name of IFF, Allyl 2-pentyloxy glycolate), FRUITATE (Trade Name of Kao Corporation, ethyl tricyclo [5.2.1.0^{2.6}]decan-2-yl carboxylate), POIRENATE (Trade Name of Kao Corporation, ethyl 2-cyclohexyl propionate), PERANAT (Trade Name of Kao Corporation, 2-methylpentyl 2-methylvalerate), MELUSAT (Trade Name of Kao Corporation, ethyl 3,5,5-trimethyl hexanoate), IROTYL (Trade Name of Kao Corporation, ethyl 2-ethylcapronate), and methyl 2,4-dihydroxy-3,6-dimethylbenzoate.

Examples of the carbonates include LIFFAROME (Trade Name of IFF, cis-3-hexenyl methyl carbonate), JASMACYCLAT (Trade Name of Kao Corporation, methyl cyclooctyl carbonate), and FLORAMAT (Trade Name of Kao Corporation, ethyl 2-tert-butylcyclohexyl carbonate).

Examples of the aldehydes include n-octanal, n-nonanal, n-decanal, n-dodecanal, 2-methyl undecanal, 10-undecenal, citronellal, citral, hydroxycitronellal, TRIPLAL (Trade Name of IFF, 2,4-dimethyl-3-cyclohexenylcarboxaldehyde), CYCLOVERTAL (Trade Name of Kao Corporation, dimethyl-3-cyclohexenyl-1-carboxaldehyde), benzaldehyde, phenylacetaldehyde, phenylpropylaldehyde, cinnamic aldehyde, dimethyltetrahydrobenzaldehyde, BOURGEONAL (Trade Name of Givaudan, 3-(p-tert-butylphenyl)-propanal), LYRAL (Trade Name of IFF, hydroxy myrac aldehyde), POLLENAL II (Trade Name of Kao Corporation, 2-cyclohexyl propanal), LILIAL (Trade Name of Givaudan, p-tert-butyl-α-methylhydrocinnamic aldehyde), p-isopropyl-α-methylhydrocinnamic aldehyde, FLORALOZONE (Trade Name of IFF, p-ethyl-α,α-dimethyl hydrocinnamic aldehyde), α-amyl cinnamic aldehyde, α-hexyl cinnamic aldehyde, heliotropine, and HELIONAL (Trade Name of IFF, alpha-methyl-1,3-benzodioxole-5-propanal).

Examples of the ketones include α-ionone, β-ionone, γ-ionone, α-methyl ionone, β-methyl ionone, γ-methyl ionone, damascenone, methyl heptenone, 4-methylene-3,5,6,6-tetramethyl-2-heptanone, acetophenone, amyl cyclopentanone, dihydrojasmone, rose ketone, carvone, menthone, camphor, acetyl cedrene, isolongifolanone, nootkatone, benzylacetone, anisylacetone, methylβ-naphthyl ketone, 2,5-dimethyl-4-hydroxy-3(2H)-furanone, maltol, muscone, civetone, cyclopentadecanone, CALONE (Trade Name of Firmenich, 7-methyl-3,5-dihydro-2H-benzodioxepin-3-one), raspberry ketone, and heliotropyl acetone.

Examples of the acetals include acetaldehyde ethylphenylpropyl acetal, citral diethyl acetal, phenylacetaldehyde glyceryl acetal, phenylacetaldehyde dimethyl acetal, ethyl acetoacetate ethylene glycol acetal, BOISAMBRENE FORTE (Trade Name of Kao Corporation), and TROENAN (Trade Name of Kao Corporation).

Examples of the ethers include cedryl methyl ether, estragole, anethole, β-naphthyl methyl ether, β-naphthyl ethyl ether, limonene oxide, rose oxide, nerol oxide, (3aα,5aβ,9aα,9bβ)]-dodecahydro-3a,6,6,9a-tetramethyl naphto[2,1-b]furan), HERBAVERT (Trade Name of Kao Corporation, 3,3,5-trimethylcyclohexyl-ethylether), and GALAXOLIDE (Trade Name of IFF, hexamethyl hexahydrocyclopentabenzopyran).

Examples of the carboxylic acids include benzoic acid, phenylacetic acid, cinnamic acid, hydrocinnamic acid, butyric acid, and 2-hexenoic acid.

Examples of the lactones include γ-decalactone, δ-decalactone, γ-valerolactone, γ-nonalactone, γ-undecalactone, δ-hexalactone, γ-jasmolactone, whisky lactone, coumarin, cyclopentadecanolide, cyclohexadecanolide, ambrettolide, 11-oxahexadecanolide, and butylidenephthalide.
Examples of the nitriles include geranyl nitrile, citronellyl nitrile, and dodecanenitrile.

Examples of the Schiff bases include aurantiol and ligantral. Examples of the natural essential oils and natural extracts include orange, lemon, lime, bergamot, vanilla, mandarin, peppermint, spearmint, lavender, chamomile, rosemary, eucalyptus, sage, basil, rose, rockrose, geranium, jasmine, ylang ylang, anise, clove, ginger, nutmeg, cardamom, cedar, cypress, olibanum, vetiver, patchouli, lemongrass, labdanum, and grapefruit.

The contents of these other fragrance materials other than Compound (I) each can be suitably selected depending on, for example, the type of blended fragrance material, the type of intended odor, and the strength of the odor but are, in the fragrance composition, preferably 0.0001% by mass or more, more preferably 0.001% by mass or more, but preferably 99.99% by mass or less, more preferably 80% by mass or less. Furthermore, the total content of other fragrance materials in the fragrance composition is preferably 5% by mass or more, more preferably 50% by mass or more, but preferably 99.99% by mass or less, more preferably 99.95% by mass or less.

When the fragrance composition of the present invention contains both Compound (I-1) and Compound (I-2) as Compound (I), the contents of other fragrance materials other than Compound (I-1) and Compound (I-2) each can be suitably selected depending on, for example, the type of blended fragrance material, the type of intended odor, and the strength of the odor. However, the contents of these in the fragrance composition each is preferably 0.0001% by mass or more, more preferably 0.001% by mass or more, but preferably 99.99% by mass or less, more preferably 80% by mass or less. Furthermore, the total content of other fragrance materials in the fragrance composition is preferably 5% by mass or more, more preferably 50% by mass or more, but preferably 99.99% by mass or less, more preferably 99.95% by mass or less.

The fragrance composition of the present invention can contain an oil, which itself has no odor, as a base that allows Compound (I) and other fragrance ingredients to be contained therein. Such an oil allows fragrance components to be blended uniformly, is easy to be blended in products, and makes it easy to impart a fragrance with a suitable strength. Specific examples of such an oil include polyhydric alcohols such as ethylene glycol, propylene glycol, butylene glycol, and dipropylene glycol, esters such as isopropyl myristate, dibutyl adipate, and diethyl sebacate, and hydrocarbons such as liquid paraffin and squalane. The content of such an oil in the fragrance composition is preferably 1% by mass or more, more preferably 10% by mass or more, and further preferably 15% by mass or more, but preferably 95% by mass or less, more preferably 80% by mass or less, and more preferably 50% by mass or less.

Furthermore, the fragrance composition of the present invention may contain a surfactant such as polyoxyethylene alkyl ether or sorbitan fatty acid ester.

### [Use as Fragrance Component]

The fragrance composition containing Compound (I) of the present invention can be used as a fragrance component for various products as a fragrance ingredient or blended fragrance material that has a natural and fresh floral feeling. Also, the fragrance composition containing Compound (I) can be used as a fragrance component for various products as a blended fragrance material having a floral feeling with fruity sweetness in addition to a natural and fresh juicyiness.

Therefore, the present invention is a method of using, as a fragrance component, a fragrance composition containing Compound (I) prepared as a fragrance material. For the method of using said fragrance composition, it can be contained, alone or in combination with other components, in the bases of toiletry products such as soaps, cosmetics, hair cosmetics, detergents, softeners, spray products, air fresheners, perfumes, and bath agents. Accordingly, the present invention is a method of using a fragrance composition containing Compound (I), a fragrance composition containing Compound (I-1) and Compound (I-2), and a fragrance composition containing Compound (I-1) prepared as fragrance materials for toiletry products such as soaps, cosmetics, hair cosmetics, detergents, softeners, spray products, air fresheners, perfumes, and bath agents.

In the method of using the fragrance composition, when the fragrance composition contains Compound (I), the amount of Compound (I) to be used is, with respect to the whole fragrance composition, preferably 0.01% by mass or more, more preferably 0.1% by mass or more, and further preferably 0.3% by mass or more, but preferably 99.99% by mass or less, more preferably 15% by mass or less, and further preferably 5% by mass or less. When Compound (I) is contained in an amount of 0.01% by mass to 99.99% by mass, a natural and fresh floral feeling can be imparted.

Furthermore, in the method of using the fragrance composition, when the fragrance composition contains Compound (I-1) and Compound (I-2), the total content of Compound (I-1) and Compound (I-2) is, with respect to the whole fragrance composition, preferably 0.01% by mass or more, more preferably 0.1% by mass or more, and further preferably 0.3% by mass or more, but preferably 99.99% by mass or less, more preferably 15% by mass or less, and further preferably 5% by mass or less. When Compound (I-1) and Compound (I-2) are contained in a total amount of 0.01% by mass to 99.99% by mass, a floral feeling with fruity sweetness can be imparted in addition to a natural and fresh juicyiness.

Particularly, it is preferable to treat hair or fibers of, for example, clothing with a fragrance composition containing Compound (I) of the present invention to impart a flower-like fragrance. Examples of the products in which it can be preferably used as the fragrance component include hair cosmetics such as shampoos and rinses as well as cleaners and softeners for clothing.

Accordingly, the present invention also provides a cleaner composition containing a fragrance composition of the present invention, a cosmetic composition containing a fragrance composition of the present invention, a fiber treatment composition containing a fragrance composition of the present invention, an aromatic air freshener containing a fragrance composition of the present invention, and a cleaning nonwoven fabric containing a fragrance composition of the present invention.

The cleaner composition of the present invention is preferably a body cleaner composition, a cleaner composition for clothing, or a cleaner composition for hard surfaces, more preferably a body cleaner composition or a cleaner composition for clothing, and further preferably a cleaner composition for clothing. Furthermore, examples of the cleaner composition of the present invention include a powder cleaner composition and a liquid cleaner composition.

Examples of the body cleaner composition include a skin cleaner composition and a hair cleaner composition, and it is preferably a hair cleaner composition.

Examples of the cleaner composition for hard surfaces include an all purpose cleaner and a dish cleaner composition.

It is preferable that the cleaner composition of the present invention contain an anionic surfactant in addition to Compound (I) or the fragrance composition containing Compound (I). Furthermore, a nonionic surfactant, a pH adjuster, a viscosity modifier, a solvent, an oil, a preservative, water, etc. can be blended therein.

The fiber treatment composition of the present invention is preferably a softener.

The cosmetic composition of the present invention is preferably a perfume, a body cosmetic, a hair cosmetic such as a shampoo or a rinse.

In the perfume of the present invention, a solvent, water, etc. can be blended therein in addition to Compound (I) or a fragrance composition containing Compound (I).

Furthermore, as described above, the cyclopropane compound represented by Formula (I) of the present invention (Compound (I)) has a natural and fresh floral odor. Therefore, Compound (I) can be used, as a fragrance ingredient or blended fragrance material having a natural and fresh floral feeling, as a fragrance component for various products. Moreover, Compound (I) can be used, as a blended fragrance material having a fresh and juicy floral feeling and fruity sweetness, as a fragrance component for various products. Thus, the present invention is a method of using Compound (I) as a fragrance component, as a fragrance material.

As a method of using said Compound (I), it can be contained, alone or in combination with other components, in the bases for toiletry products such as soaps, cosmetics, hair cosmetics, detergents, softeners, spray products, air fresheners, perfumes, and bath agents. Accordingly, the present invention is a method of using Compound (I), both Compound (I-1) and Compound (I-2), or Compound (I-1) as a fragrance material for toiletry products such as soaps, cosmetics, hair cosmetics, detergents, softeners, spray products, air fresheners, perfumes, and bath agents.

With respect to the embodiments described above, the present invention further discloses the following fragrance compositions, production methods, method of use, and uses.
<1> A cyclopropane compound represented by Formula (I). 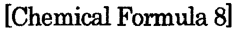 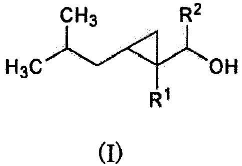
   [In Formula (I), R¹ is a methyl group and R² is a methyl group, or R¹ is a hydrogen atom and R² is an ethyl group.]
<2> The cyclopropane compound according to item <1>, wherein R¹ is a methyl group and R² is a methyl group.
<3> A fragrance composition, containing a cyclopropane compound according to item <1> or <2>.
<4> A fragrance composition, the content of a cyclopropane compound according to item <1> or <2> in the fragrance composition being preferably 0.01% by mass or more, more preferably 0.1% by mass or more, and further preferably 0.3% by mass or more, but preferably 99.99% by mass or less, more preferably 15% by mass or less, and further preferably 5% by mass or less.
<5> A fragrance composition, containing a cyclopropane compound according to item <1> where R¹ is a methyl group and R² is a methyl group, and a cyclopropane compound according to item <1> where R¹ is a hydrogen atom and R² is an ethyl group.
<6> The fragrance composition according to item <5>, wherein the mass ratio of a cyclopropane compound according to item <1> where R¹ is a methyl group and R² is a methyl group to a cyclopropane compound according to item <1> where R¹ is a hydrogen atom and R² is an ethyl group, i.e., (a cyclopropane compound according to item <1> where R¹ is a methyl group and R² is a methyl group)/(a cyclopropane compound according to item <1> where R¹ is a hydrogen atom and R² is an ethyl group, is preferably 70/30 or more, more preferably 80/20 or more, and further preferably 90/10 or more, but preferably 99.9/0.1 or less, more preferably 99/1 or less, further preferably 98/2 or less, and still further preferably 95/5 or less.
<7> The fragrance composition according to item <5> or <6>, wherein the mass ratio of a cyclopropane compound according to item <1> where R¹ is a methyl group and R² is a methyl group to a cyclopropane compound according to item <1> where R¹ is a hydrogen atom and R² is an ethyl group, i.e., (the cyclopropane compound according to item <1> where R¹ is a methyl group and R² is a methyl group)/(the cyclopropane compound according to item <1> where R¹ is a hydrogen atom and R² is an ethyl group), is preferably 70/30 to 99.9/0.1, more preferably 80/20 to 99/1, further preferably 90/10 to 98/2, and still further preferably 90/10 to 95/5.
<8> The fragrance composition according to any one of items <5> to <7>, wherein the total content of a cyclopropane compound according to item <1> wherein R¹ is a methyl group and R² is a methyl group and a cyclopropane compound according to item <1> wherein R¹ is a hydrogen atom and R² is an ethyl group is preferably 0.01% by mass or more, more preferably 0.1% by mass or more, and further preferably 0.3% by mass or more, but preferably 99.99% by mass or less, more preferably 15% by mass or less, and further preferably 5% by mass or less.
<9> The fragrance composition according to any one of items <3> to <8>, further containing one or more fragrance components selected from the group consisting of alcohols, hydrocarbons, phenols, esters, carbonates, aldehydes, ketones, acetals, ethers, carboxylic acids, lactones, nitriles, Schiff bases, natural essential oils, and natural extracts other than the cyclopropane compound represented by Formula (I).
<10> The fragrance composition according to any one of items <3> to <9>, further containing one or more other fragrance materials selected from the group consisting of alcohols, hydrocarbons, phenols, esters, aldehydes, ketones, acetals, and ethers other than the cyclopropane compound represented by Formula (I), preferably another fragrance material of alcohols and/or esters.
<11> The fragrance composition according to any one of items <3> to <10>, wherein the contents of the other fragrance materials in the fragrance composition each is preferably 0.0001% by mass or more, more preferably 0.001% by mass or more, but preferably 99.99% by mass or less, more preferably 80% by mass or less.
<12> The fragrance composition according to any one of items <3> to <12>, wherein the total content of the other fragrance materials in the fragrance composition is preferably 5% by mass or more, more preferably 50% by mass or more, but preferably 99.99% by mass or less, more preferably 99.95% by mass or less.
<13> The fragrance composition according to any one of items <3> to <12>, further containing an oil, preferably polyhydric alcohols (more preferably ethylene glycol, propylene glycol, butylene glycol, or dipropylene glycol), esters (more preferably isopropyl myristate, dibutyl adipate, or diethyl sebacate), or hydrocarbons (more preferably liquid paraffin or squalane).
<14> The fragrance composition according to any one of items <3> to <13>, wherein the content of the oil in the fragrance composition is preferably 1% by mass or more, more preferably 10% by mass or more, and further preferably 15% by mass or more, but preferably 95% by mass or less, more preferably 80% by mass or less, and further preferably 50% by mass or less.
<15> A fiber treatment composition, containing a fragrance composition according to any one of items <3> to <14>.
<16> A softener, containing a fragrance composition according to any one of items <3> to <14>.
<17> A cleaner composition (preferably a body cleaner composition, a cleaner composition for clothing, or a cleaner composition for hard surfaces, more preferably a body cleaner composition or a cleaner composition for clothing, and further preferably a cleaner composition for clothing), containing a fragrance composition according to any one of items <3> to <14>.
<18> A cosmetic composition (preferably a perfume or a body cosmetic), containing a fragrance composition according to any one of items <3> to <14>.
<19> A hair cosmetic (preferably a shampoo or a rinse), containing a fragrance composition according to any one of items <3> to <14>.
<20> A method of using a cyclopropane compound according to item <1> or <2> as a fragrance component.
<21> A method of producing a cyclopropane compound represented by Formula (I), including cyclopropanating a compound represented by Formula (II) using iodomethane (preferably, using iodomethane in a solvent in the presence of a metal catalyst such as a zinc catalyst (more preferably a zinc catalyst, further preferably dialkylzinc such as dimethylzinc or diethylzinc) or under ultrasonic irradiation) to obtain a cyclopropane compound represented by Formula (1). 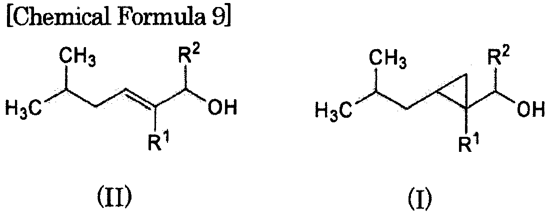
   [In Formula (I) and Formula (II), R¹ is a methyl group and R² is a methyl group, or R¹ is a hydrogen atom and R² is an ethyl group.]
<22> A method of producing a cyclopropane compound represented by Formula (I), including reacting a compound represented by Formula (II) with diazomethane in the presence of lead acetate to cyclopropanate it and thereby obtaining a cyclopropane compound represented by Formula (I). 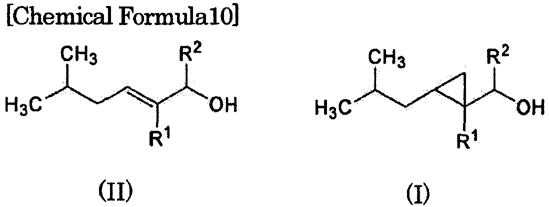
   [In Formula (I) and Formula (II), R¹ is a methyl group and R² is a methyl group, or R¹ is a hydrogen atom and R² is an ethyl group.]
<23> Use of a fragrance composition according to any one of items <3> to <14> as a fragrance component.
<24> Use of a cyclopropane compound represented by Formula (I) according to item <1> or <2> as a fragrance material.
<25> The use according to item <24> of a cyclopropane compound represented by Formula (I) according to item <1> or <2> as a fragrance material for a fiber treatment composition, a softener, a cleaner composition (preferably a body cleaner composition, a cleaner composition for clothing, or a cleaner composition for hard surfaces, more preferably a body cleaner composition or a cleaner composition for clothing, and further preferably a cleaner composition for clothing), a cosmetic composition (preferably a perfume or a body cosmetic), or a hair cosmetic (preferably a shampoo or a rinse).
<26> A method of producing a composition selected from the group consisting of a fiber treatment composition, a softener, a cleaner composition (preferably a body cleaner composition, a cleaner composition for clothing, or a cleaner composition for hard surfaces, more preferably a body cleaner composition or a cleaner composition for clothing, and further preferably a cleaner composition for clothing), a cosmetic composition (preferably, a perfume or a body cosmetic), and a hair cosmetic (preferably a shampoo or a rinse), the method being a method of adding a cyclopropane compound represented by Formula (I) according to item <1> or <2> to said composition.

### [Examples]

### [Identification of Compounds]

The structure of each compound obtained in the following production examples was identified by nuclear magnetic resonance spectrum (¹H-NMR). The nuclear magnetic resonance spectra were measured with "Mercury 400" (product name) manufactured by Varian using chloroform-d as a solvent.

### [Purity of Compounds]

The purity of each compound obtained in the following production examples was measured with a gas chromatograph (column: non-polar column (30 m), carrier gas: He, split analysis).

### [Production Example 1]

### Production of 3,6-dimethylhept-3-en-2-one (Compound (III-1)) and 7-methyloct-4-en-3-one (Compound (III-2))

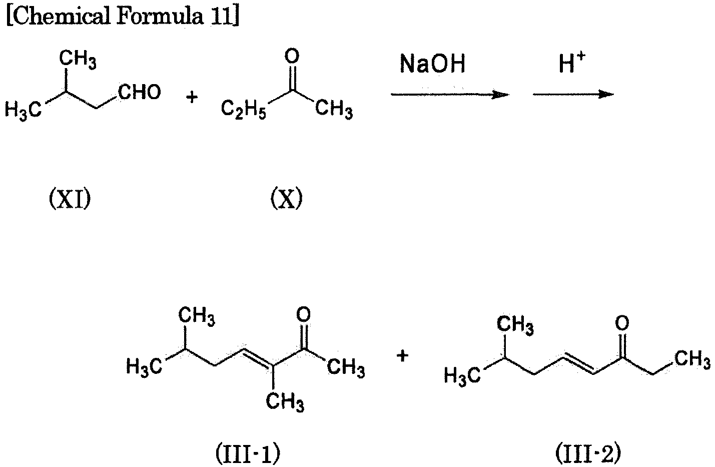

Sodium hydroxide (0.5 g), water (100 g), and 2-butanone (X) (210 g, 2.9 mol) were placed in a flask, which was maintained at 15°C. While the mixture was stirred, isovaleraldehyde (XI) (100 g, 1.2 mol) was added dropwise over five hours and then sodium hydroxide (1.0 g) was additionally added. This was further stirred at 25°C for 48 hours. After stirring, the reaction solution was allowed to stand and then the lower layer was removed. Thereafter, excessive 2-butanone (X) was evaporated to dryness from the upper layer.

Subsequently, 85% phosphoric acid (5 g) was added to the upper layer portion of the reaction solution, and the flask was fitted with a water fractionator and heated to 120°C to perform dehydration. Sodium hydroxide (3.5 g) was added to the reaction solution obtained after the dehydration to neutralize it and then it was dried. The resultant reaction solution was concentrated and thereby a residue (105 g) was obtained.

The residue thus obtained was purified using a silica gel column (developing solvent: hexane/ethyl acetate = 99/1 (v / v)). The purified product thus obtained was further purified by distillation. Thus, 55 g of Compound (III-1) and 10 g of Compound (III-2) were obtained.

The purity of the resultant Compound (III-1) was 97% and the purity of Compound (III-2) was 94%.

### [Production Example 2]

### Production of 3,6-dimethylheptene-2-ol (Compound (II-1))

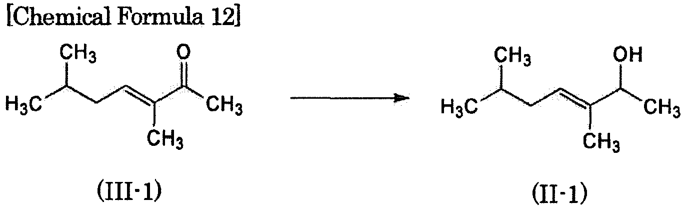

3,6-dimethylhept-3-en-2-one (Compound (III-1)) (6.1 g, 0.044 mol), cerium chloride heptahydrate (1.7 g, 0.044 mol), and methanol (45 mL) were added to a flask under a nitrogen atmosphere and cooled to 0°C. The mixture was further cooled to -8°C and then sodium borohydride (1.7 g, 0.044 mol) was added in portions to the reaction mixture over 15 minutes. After stirring for two hours, the reaction solution was brought to 25°C and an aqueous sulfuric acid solution was added thereto, which then was stirred for 20 minutes. Thereafter, an aqueous sodium hydroxide solution was added to the reaction mixture. After the pH of the reaction mixture was confirmed to be 6 to 7 using pH test paper, methanol was evaporated to dryness from the reaction mixture. Tert-butyl methyl ether and saturated brine were added to and mixed with the reaction mixture and then the aqueous layer and the organic layer were separated from each other. After tert-butyl methyl ether was added to and mixed with the aqueous layer, the aqueous layer and the organic layer were separated from each other again. Saturated brine was added to and mixed with the organic layer and then the aqueous layer was removed. The tert-butyl methyl ether was evaporated to dryness from the organic layer and thereby 2.1 g of 3,6-dimethylheptene-2-ol (Compound (II-1)) (purity 96%) was obtained in a crude yield of 76%.

### [Example 1]

### Production of 1-(2-isobutyl-1-methylcyclopropyl)ethan-1-ol (Compound (I-1))

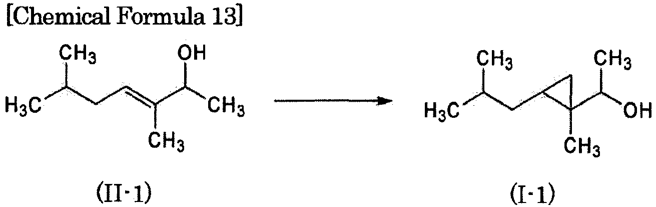

3,6-Dimethylheptene-2-ol (Compound (II-1)) (3.2 g, 0.22 mol) and dichloromethane (45 mL) were added to a flask and cooled to -10°C. A solution of diethylzinc in hexane (1.09 mol/L, 50 mL, 0.055 mol, 0.25 times by mole with respect to Compound (II-1)) was added to the reaction mixture, which then was stirred for 30 minutes. Thereafter, diiodomethane (14.6 g, 0.055 mol) was slowly added dropwise using a dropping funnel. After completion of the dropwise addition, the reaction solution was brought to 25°C and stirred for two hours. After 20 mL of saturated aqueous ammonium chloride solution was added to the reaction mixture, 30 mL of diethyl ether and 35 mL of aqueous 2N hydrochloric acid solution were added thereto, which then was stirred until the aqueous layer and the organic layer became transparent. After the organic layer and the aqueous layer were separated from each other, water was added to and mixed with the organic layer and then the aqueous layer was removed. After the organic layer was dried over sodium sulfate, dichloromethane was evaporated to dryness. In the resultant residue, the product was separated using silica gel column chromatography (developing solvent: hexane/ethyl acetate = 80/20 (v / v)) and thereby 2.1 g (0.013 mol) of 1-(2-isobutyl-1-methylcyclopropyl)ethan-1-ol (Compound (I-1)) was obtained in a yield of 62%.

The measurement results of ¹H-NMR and the odor evaluations of Compound (I-1) are described below.
¹H-NMR (CDCl₃, 400 MHz, δppm): -0.02-0.02 (m, 1H), 0.54-0.60 (m, 2H), 0.92 (d, J=7.2 Hz, 3H), 0.94 (d, J=6.8 Hz, 3H), 1.03 (s, 3H), 1.08-1.16 (m, 1H), 1.20 (d, J=6.4 Hz, 3H), 1.26-1.34 (m, 1H), 1.40-1.44 (m, 1H), 1.56-1.67 (m, 1H), 2.99 (dq, J=6.4 Hz, 3H)

### [Production Example 3]

### Production of 7-methyloct-4-en-3-ol (Compound (II-2))

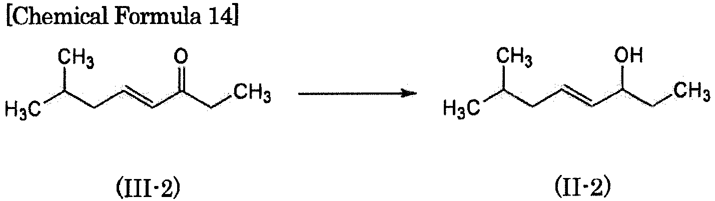

7-methyloct-4-en-3-one (Compound (III-2)) (4.0 g, 0.032 mol), cerium chloride heptahydrate (11.8 g, 0.032 mol), and methanol (30 mL) were added to a flask under a nitrogen atmosphere and stirred at room temperature. Furthermore, the mixture was cooled to -10°C and then sodium borohydride (1.2 g, 0.032 mol) was added in portions to the reaction mixture over 20 minutes. After stirring for two hours, the reaction solution was brought to 25°C and an aqueous sulfuric acid solution was added thereto, which then was stirred for 20 minutes. Thereafter, an aqueous sodium hydroxide solution was added to the reaction mixture. After the pH of the reaction mixture was confirmed to be 6 to 7 using pH test paper, methanol was evaporated to dryness. Diethyl ether and saturated brine were added to and mixed with the reaction mixture and then the aqueous layer and the organic layer were separated from each other. After diethyl ether was added to and mixed with the aqueous layer, the aqueous layer and the organic layer were separated from each other again. Saturated brine was added to and mixed with the organic layer and then the aqueous layer was removed. The diethyl ether was evaporated to dryness from the organic layer and thereby 3.7 g of 7-methyloct-4-en-3-ol (Compound (II-2)) (purity 97%) was obtained in a crude yield of 88%.

### [Example 2]

### Production of 1-(2-isobutylcyclopropyl)propan-1-ol (Compound (I-2))

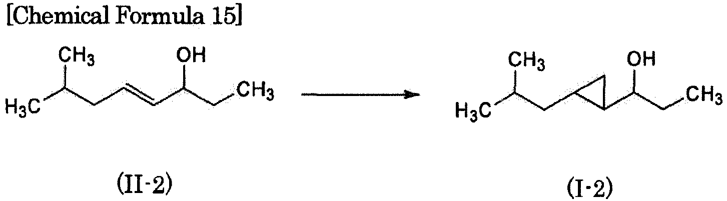

7-methyloct-4-en-3-ol (Compound (II-2)) (1.7 g, 0.013 mol) and dichloromethane (60 mL) were added to a flask and cooled to -10°C. A solution of diethylzinc in hexane (1.09 mol/L, 30 mL, 0.033 mol, 2.5 times by mole with respect to Compound (II-2)) was added thereto, which then was stirred for 15 minutes. Thereafter, diiodomethane (8.8 g, 0.033 mol) was slowly added dropwise using a dropping funnel. After completion of the dropwise addition, the reaction solution was brought to 25°C and stirred for 11 hours. After 10 mL of saturated aqueous ammonium chloride solution was added to the reaction mixture, 80 mL of diethyl ether and 25 mL of aqueous 2N hydrochloric acid solution were added thereto, which then was stirred until the aqueous layer and the organic layer became transparent. After the organic layer and the aqueous layer were separated from each other, water was added to and mixed with the organic layer and then the aqueous layer was removed. After the organic layer was dried over sodium sulfate, dichloromethane was evaporated to dryness. In the resultant residue, the product was separated using silica gel column chromatography (developing solvent: hexane/ethyl acetate = 85/15 (v / v)) and thereby 1.5 g (0.010 mol) of 1- (2-isobutylcyclopropyl)propan-1-ol (Compound (I-2)) was obtained in a yield of 79%.

The measurement results of ¹H-NMR and the odor evaluations of Compound (I-2) are described below.
¹H-NMR (CDCl₃, 400 MHz, δppm): 0.25-0.30 (m, 1H), 0.42-0.48 (m, 1H), 0.59-0.69 (m, 2H), 0.91 (d, J=6.4 Hz, 3H), 0.92 (d, J=6.8 Hz, 3H), 0.95-1.07 (m, 1H), 0.99 (d, J=7.2 Hz, 3H), 1.18-1.27 (m, 1H), 1.43 (d, J=2.8 Hz, 1H), 1.55-1.71 (m, 1H), 2.18-2.88 (m, 1H)

### [Comparative Example 1]

### Production of 4- (2,2-dimethylcyclopropyl)butan-2-ol (may referred to as Compound (XX) in the present specification)

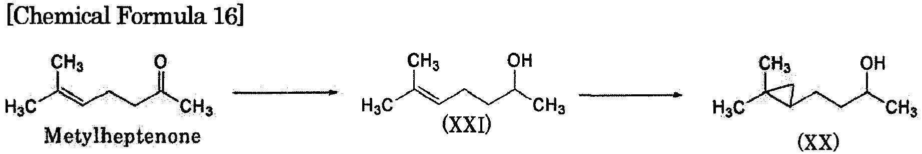

After methyl heptenone (6.3 g, 0.050 mol) and methanol (50 mL) were added to a flask and cooled to -10°C, sodium borohydride (1.0 g, 0.025 mol) was added in portions to the reaction mixture over 20 minutes. After stirring for two hours, the reaction solution was brought to 25°C and an aqueous sulfuric acid solution was added thereto, which then was stirred for 20 minutes. Thereafter, an aqueous sodium hydroxide solution was added to the reaction mixture. After the pH of the reaction mixture was confirmed to be 6 to 7 using pH test paper, methanol was evaporated to dryness. Diethyl ether and saturated brine were added thereto and mixed therewith and then the aqueous layer and the organic layer were separated from each other. After diethyl methyl ether was added to and mixed with the aqueous layer, the aqueous layer and the organic layer were separated from each other again. Saturated brine was added to and mixed with the organic layer and then the aqueous layer was removed. The diethyl ether was evaporated to dryness from the organic layer and thereby 5.8 g of 6-methylhept-5-en-2-ol (Compound (XXI)) (purity 97%) was obtained in a crude yield of 88%.

6-Methylhept-5-en-2-ol (Compound (XXI)) (2.9 g, 0.022 mol) and dichloromethane (45 mL) were added to a flask and cooled to -10°C. A solution of diethylzinc in hexane (1.09 mol/L, 50 mL, 0.055 mol) was added thereto, which then was stirred for 30 minutes. Thereafter, diiodomethane (14.6 g, 0.055 mol) was slowly added dropwise using a dropping funnel. After completion of the dropwise addition, the reaction solution was brought to 25°C and stirred for 20 hours. Thereafter, the reaction mixture was stirred at 40°C for 3.5 hours. After 20 mL of saturated aqueous ammonium chloride solution was added thereto, 100 mL of diethyl ether and 25 mL of aqueous 2N hydrochloric acid solution were added thereto, and the mixture was stirred until the aqueous layer and the organic layer became transparent. After the organic layer and the aqueous layer were separated from each other, water was added to and mixed with the organic layer, and then the aqueous layer was removed. After the organic layer was dried over sodium sulfate, dichloromethane and diethyl ether were evaporated to dryness. In the resultant residue, the product was separated using silica gel column chromatography (developing solvent: hexane/ethyl acetate = 80/20 (v / v)) and thereby 2.3 g of 4- (2,2-dimethylcyclopropyl)butan-2-ol (Compound (XX)) was obtained in a yield of 72%.

The measurement results of ¹H-NMR and the odor evaluations of Compound (XX) are described below. ¹H-NMR (CDCl₃, 400 MHz, δppm: -0.16-0.08 (m, 1H), 0.32-0.40 (m, 1H), 0.42-0.52 (m, 1H), 1.03 (d, J=7.6 Hz, 6H), 1.20 (d, J=6.4 Hz, 3H), 1.21-1.62 (m, 5H), 3.78-3.88 (m, 1H)

### [Odor Evaluation of Examples 1 to 3 and Comparative Example 1]

Compound (I-1) obtained in Example 1, Compound (I-2) obtained in Example 2, and a mixture of Compound (I-1) and Compound (I-2) (described as Example 3) as well as Compound (XX) obtained in Comparative Example 1 were subjected to odor evaluation according to the following odor evaluation method. The results thus obtained are shown in Table 1.

### [Odor Evaluation Method]

Two experts who had an experience of five years of blending odors and evaluating fragrance materials determined the fragrance notes by a smelling strip method. About 5 mm of the end of each smelling strip (fragrance test paper with a width of 6 mm and a length of 150 mm) was immersed in a sample and thereby evaluation was performed.

For the odor evaluation performed using the powder detergents for clothing of Examples 4 to 6 and Comparative Examples 2 and 3, 4 L of water was put in a tub, 3 g of the prepared powder detergent was dissolved therein, and then the two experts evaluated the odor directly to judge the fragrance note.

With respect to the odors, the fragrance mainly felt (main odor) was described, and furthermore, the fragrances secondarily felt (secondary odors) were noted. In addition, the floral feeling was also determined.

**[Table 1]**

| Fragrance Materials | Examples | | | C. Example |
|---|---|---|---|---|
| | 1 | 2 | 3 | 1 |
| Compound (I-1) | 100 | - | 95 | - |
| Compound (I-2) | - | 100 | 5 | - |
| Compound (XX) | - | - | - | 100 |
| Main Odor | Floral | Floral | Floral | Citrus |
| Secondary Odors | Citrus | Herbal | Fruity | Floral |
| | | Fruity | Citrus | Fruity |
| Floral Feeling | Felt Well (Lilac, Rose) | Felt Well (Slightly Muguet) | Felt Well (Lilac) | Slightly Felt |

As shown in Table 1 above, the cyclopropane compound of the present invention was confirmed to have a main odor with a floral note.

### [Odor Evaluation of Examples 4 to 6 and Comparative Examples 2 and 3]

Using Compound (I-1) obtained in Example 1, Compound (I-2) obtained in Example 2, the mixture of Compound (I-1) and Compound (I-2) (Example 3), and Compound (XX) obtained in Comparative Example 1, fragrance compositions for white floral type detergents for clothing having the compositions indicated in Table 2 were prepared. These fragrance compositions each was blended in the preparation example of a detergent for clothing shown in Table 3, which were used as samples for odor evaluation to be performed. They were judged by sensory evaluation for the case of using each of them for a powder detergent for clothing. The results of the sensory evaluation are shown in Table 4.

**[Table 2]**

| | C. Ex. 2 | Ex. 4 | Ex. 5 | Ex. 6 | C. Ex. 3 |
|---|---|---|---|---|---|
| Dihydromyrcenol | 50 | 50 | 50 | 50 | 50 |
| TRIPLAL¹⁾ | 4 | 4 | 4 | 4 | 4 |
| MAGNOL²⁾ | 2 | 2 | 2 | 2 | 2 |
| UNDECAVERTOL³⁾ | 2 | 2 | 2 | 2 | 2 |
| o-tert-Butylcyclohexyl Acetate | 20 | 20 | 20 | 20 | 20 |
| POIRENATE⁴⁾ | 2 | 2 | 2 | 2 | 2 |
| Raspberry Ketone | 5 | 5 | 5 | 5 | 5 |
| FRUITATE⁵⁾ | 2 | 2 | 2 | 2 | 2 |
| Tricyclodecenyl Propionate | 100 | 100 | 100 | 100 | 100 |
| Geraniol | 100 | 100 | 100 | 100 | 100 |
| Rose Oxide | 1 | 1 | 1 | 1 | 1 |
| α-Hexyl Cinnamic Aldehyde | 100 | 100 | 100 | 100 | 100 |
| Methyl Dihydrojasmonate | 50 | 50 | 50 | 50 | 50 |
| Dimethyl Anthranilate | 5 | 5 | 5 | 5 | 5 |
| Methyl-β-Naphthyl Ketone | 5 | 5 | 5 | 5 | 5 |
| LILIAL⁶⁾ | 100 | 100 | 100 | 100 | 100 |
| Terpineol | 50 | 50 | 50 | 50 | 50 |
| Phenylacetaldehyde Dimethyl Acetal | 4 | 4 | 4 | 4 | 4 |
| AMBER CORE⁷⁾ | 30 | 30 | 30 | 30 | 30 |
| Isobornylcyclohexanol | 20 | 20 | 20 | 20 | 20 |
| GALAXOLIDE⁸⁾ 50% IPM⁹⁾ | 60 | 60 | 60 | 60 | 60 |
| AMBROXAN¹⁰⁾ 5% DPG¹¹⁾ | 4 | 4 | 4 | 4 | 4 |
| Heliotropyl Acetone | 1 | 1 | 1 | 1 | 1 |
| Compound (I-1) | - | 50 | - | 47.5 | - |
| Compound (I-2) | - | - | 50 | 2.5 | - |
| Compound (XX) | - | - | - | - | 50 |
| Dipropylene Glycol | 283 | 233 | 233 | 233 | 233 |
| Total | 1000 | 1000 | 1000 | 1000 | 1000 |

1) TRIPLAL: IFF (Trade Name), Compound Name:
   2,4 -dimethyl-3-cyclohexene-1-carboxaldehyde
2) MAGNOL: Kao Corporation (Trade Name), a mixture containing ethyl norbonyl cyclohexanol as a main component
3) UNDECAVERTOL: Givaudan (Trade Name), Compound Name:
   4-Methyl-3-decene-5-ol
4) POIRENATE: Kao Corporation (Trade Name), Compound Name: Ethyl 2-cyclohexyl propionate
5) FRUITATE: Kao Corporation (Trade Name), Compound Name: Ethyl tricyclo [5.2.1.0^{2,6}]decan-2-yl carboxylate
6) LILIAL: Givaudan (Trade Name), Compound Name:
   p-tert-Butyl-α-methylhydrocinnamic aldehyde
7) AMBER CORE: Kao Corporation (Trade Name), Compound Name: 1-(2-tert-butyl Cyclohexyloxy)-2-butanol
8) GALAXOLIDE: IFF (Trade Name), Compound Name: Hexamethyl hexahydrocyclopentabenzopyran
9) 50% IPM: denotes 50% by mass isopropyl myristate (IPM) solution.
10) AMBROXAN: Kao Corporation (Trade Name), Compound Name: [3aR-(3aα,5aβ,9aα,9bβ)]-Dodecahydro-3a,6,6,9a-tetramethyl naphto[2,1-b]furan 11) 5% DPG: denotes 5% by mass dipropylene glycol solution.

**[Table 3]**

| Powder Detergent for Clothing | Blending Amount (% by mass) |
|---|---|
| Sodium Linear Alkylbenzene Sulfonate | 40 |
| Sodium Tallowate | 1 |
| Polyoxyethylene Alkyl Ether | 5 |
| Zeolite | 22.5 |
| Sodium Carbonate | 10 |
| Fluorescent Dye | 0.5 |
| Fragrance Composition of One of Examples 4 to 6 and Comparative Examples 2 and 3 | 0.5 |
| Mirabilite | Proper Amount |

**[Table 4]**

| | |
|---|---|
| C. Example 2 | Gorgeous white floral fragrance |
| Example 4 | Gorgeous white floral fragrance plus juicyiness, with increased brightness |
| Example 5 | Gorgeous white floral fragrance plus green feeling |
| Example 6 | Gorgeous white floral fragrance plus juicyiness and green feeling |
| C. Example 3 | White floral fragrance but with heavy impression Less gorgeous |

As shown in Table 4 above, when the cyclopropane compound of the present invention was used as the fragrance component, natural and fresh floral notes were confirmed to be obtained.

### [Odor Evaluation of Examples 7 to 9 and Comparative Examples 4 and 5]

Using Compound (I-1) obtained in Example 1**,** Compound (I-2) obtained in Example 2, the mixture of Compound (I-1) and Compound (I-2) (Example 3), and Compound (XX) obtained in Comparative Example 1, fragrance compositions for floral-type softeners having the compositions shown in Table 5 were prepared. These fragrance compositions each were blended in the preparation example of a softener shown in Table 6, which were used as samples for odor evaluation to be performed in the same manner as in Example 1. They were judged by sensory evaluation for the case of using each of them for a softener. The results of the sensory evaluation are shown in Table 7.

**[Table 5]**

| | C. Ex. 4 | Ex. 7 | Ex. 8 | Ex. 9 | C. Ex. 5 |
|---|---|---|---|---|---|
| ALLYLAMYL GLYCOLATE¹⁾ | 5 | 5 | 5 | 5 | 5 |
| Citronellol | 50 | 50 | 50 | 50 | 50 |
| Heliotropine | 50 | 50 | 50 | 50 | 50 |
| α-Hexyl Cinnamic Aldehyde | 200 | 200 | 200 | 200 | 200 |
| LILIAL²⁾ | 100 | 100 | 100 | 100 | 100 |
| Limonene | 50 | 50 | 50 | 50 | 50 |
| γ-Methyl Ionone | 50 | 50 | 50 | 50 | 50 |
| Methyl 2,4-Dihydroxy-3,6-Dimethylbenzoate | 5 | 5 | 5 | 5 | 5 |
| p-tert-Butylcyclohexyl Acetate | 50 | 50 | 50 | 50 | 50 |
| Phenylethyl Alcohol | 100 | 100 | 100 | 100 | 100 |
| FRUITATE³⁾ | 20 | 20 | 20 | 20 | 20 |
| GALAXOLIDE⁴⁾ | 100 | 100 | 100 | 100 | 100 |
| Olibanum Resinoid 50% DPG⁵⁾ | 50 | 50 | 50 | 50 | 50 |
| Compound (I-1) | - | 50 | - | 47.5 | - |
| Compound (I-2) | - | - | 50 | 2.5 | - |
| Compound (XX) | - | - | - | - | 50 |
| Dipropylene Glycol | 170 | 120 | 120 | 120 | 120 |
| Total | 1000 | 1000 | 1000 | 1000 | 1000 |

| | | | | | |
|---|---|---|---|---|---|
| 1) ALLYL AMYL GLYCOLATE: IFF (Trade Name), Compound Name: Iso-amyl oxyacetic acid allylester 2) LILIAL: Givaudan (Trade Name), Compound Name: p-tert-Butyl-α-methylhydrocinnamic aldehyde 3) FRUITATE: Kao Corporation (Trade Name), Compound Name: Ethyl tricyclo [5.2.1.0^{2,6}] decan-2 carboxylate 4)GALAXOLIDE: IFF (Trade Name), Compound Name: 1,3,4,6,7,8-Hexahydro-4,6,6,7,8,8-hexamethylcyclopenta-γ-2-benzopyran 5) 50% DPG: denotes 50% by mass dipropylene glycol (DPG) solution. | | | | | |

**[Table 6]**

| Softener | Blending Amount (% by mass) |
|---|---|
| N,N-Di[2-(alkanoyloxy)ethyl]-N-(2-hydroxyethyl)-N-methylammonium sulfate | 15 |
| Stearic Acid | 0.5 |
| Calcium Chloride | 0.05 |
| Ethanol | 2 |
| Fragrance Composition of One of Examples 7 to 9 and Comparative Examples 4 and 5 | 0.5 |
| Ion Exchanged Water | Proper Amount |
| pH Adjuster¹⁾ | Proper Amount |

| | |
|---|---|
| 1) Adjusted to obtain a pH of 4. | |

**[Table 7]**

| | |
|---|---|
| C. Example 4 | Floral-like odor |
| Example 7 | Floral-like odor, with freshness and juicyiness increased |
| Example 8 | Floral-like odor, with fruity feeling added |
| Example 9 | Floral-like odor, with bright fruity feeling added |
| C. Example 5 | Floral-like odor, but with earthy-like odor and gloomy impression |

As shown in Table 7 above, when the cyclopropane compound of the present invention was used as the fragrance component, a natural and fresh floral feeling was confirmed to be obtained.

### [Odor Evaluation of Examples 10 to 12 and Comparative Examples 6 and 7]

Using Compound (I-1) obtained in Example 1, Compound (I-2) obtained in Example 2, the mixture of Compound (I-1) and Compound (I-2) (Example 3), and Compound (XX) obtained in Comparative Example 1, fragrance compositions for rose-type shampoos having the compositions shown in Table 8 were prepared. These fragrance compositions each were blended in the preparation example of a shampoo shown in Table 9, which were used as samples for odor evaluation to be performed in the same manner as in Example 1. They were judged by sensory evaluation for the case of using each of them for a shampoo. The results of the sensory evaluation are shown in Table 10.

**[Table 8]**

| | C. Ex. 6 | Ex 10 | Ex. 11 | Ex. 12 | C. Ex. 7 |
|---|---|---|---|---|---|
| N-Nonanal 10% DPG¹⁾ | 10 | 10 | 10 | 10 | 10 |
| Eugenol | 15 | 15 | 15 | 15 | 15 |
| Damascenone 10% DPG¹⁾ | 3 | 3 | 3 | 3 | 3 |
| Geraniol | 150 | 150 | 150 | 150 | 150 |
| Nerol | 20 | 20 | 20 | 20 | 20 |
| Phenylethyl Acetate | 5 | 5 | 5 | 5 | 5 |
| Phenylethyl Alcohol | 500 | 500 | 500 | 500 | 500 |
| Rose Oxide 10% DPG¹⁾ | 10 | 10 | 10 | 10 | 10 |
| Compound (I-1) | - | 50 | - | 47.5 | - |
| Compound (I-2) | - | - | 50 | 2.5 | - |
| Compound (XX) | - | - | - | - | 50 |
| Dipropylene Glycol | 287 | 237 | 237 | 237 | 237 |
| Total | 1000 | 1000 | 1000 | 1000 | 1000 |

| | | | | | |
|---|---|---|---|---|---|
| 1)10% DPG: denotes 10% by mass dipropylene glycol solution. | | | | | |

**[Table 9]**

| Shampoo | Blending Amount (% by mass) |
|---|---|
| cis-1,2-Cyclohexanedicarboxylic Acid | 1 |
| Sodium Polyoxyethylene (3) Lauryl Ether Sulfate | 10 |
| Sodium Lauryl Sulfate | 5 |
| Polyoxyethylene (15) Lauryl Ether | 1 |
| Lauric Acid Diethanolamide | 1 |
| Lauramidopropyl Betaine | 1 |
| Stearyltrimethylammonium Chloride | 0.1 |
| Fragrance Composition of One of Examples 10 to 12 and Comparative Examples 6 and 7 | 1 |
| Purified Water | Proper Amount |

**[Table 10]**

| | |
|---|---|
| C. Example 6 | Rose-like odor |
| Example 10 | Rose-like odor, with natural feeling, juicyiness, and soft impression added. |
| Example 11 | Rose-like odor, with green feeling added. |
| Example 12 | Rose-like odor, with soft impression and green feeling added. |
| C. Example 7 | Rose-like odor was reduced in sweetness and softness, with less rose feeling. |

As shown in Table 10 above, when the cyclopropane compound of the present invention was used as the fragrance component, natural and fresh floral notes were confirmed to be obtained.

### [Industrial Applicability]

The cyclopropane compound of the present invention can be used as a fragrance ingredient with a natural and fresh floral fragrance note. It can be used, alone or in combination with other components, as a fragrance component for cleaners and softeners for clothing as well as toiletry products such as hair cosmetics.

## Claims

1. A cyclopropane compound represented by Formula (I): 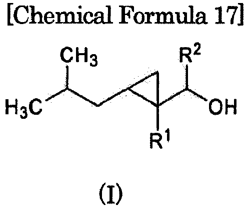 wherein R¹ is a methyl group and R² is a methyl group, or R¹ is a hydrogen atom and R² is an ethyl group.

2. The cyclopropane compound according to claim 1, wherein R¹ is a methyl group and R² is a methyl group.

3. A fragrance composition comprising a cyclopropane compound according to claim 1 or 2.

4. A fragrance composition comprising a cyclopropane compound according to claim 1 wherein R¹ is a methyl group and R² is a methyl group, and a cyclopropane compound according to claim 1 wherein R¹ is a hydrogen atom and R² is an ethyl group.

5. The fragrance composition according to claim 4, wherein the mass ratio of a cyclopropane compound according to claim 1 wherein R¹ is a methyl group and R² is a methyl group to a cyclopropane compound according to claim 1 wherein R¹ is a hydrogen atom and R² is an ethyl group (the cyclopropane compound according to claim 1 wherein R¹ is a methyl group and R² is a methyl group)/(the cyclopropane compound according to claim 1 wherein R¹ is a hydrogen atom and R² is an ethyl group) is 70/30 to 99.9/0.1.

6. The fragrance composition according to any one of claims 3 to 5, further comprising one or more other fragrance materials selected from the group consisting of alcohols, hydrocarbons, phenols, esters, aldehydes, ketones, acetals, and ethers other than the cyclopropane compound represented by Formula (I).

7. The fragrance composition according to any one of claims 3 to 6, further comprising an oil.

8. A fiber treatment composition comprising a fragrance composition according to any one of claims 3 to 7.

9. A softener comprising a fragrance composition according to any one of claims 3 to 7.

10. A cleaner composition comprising a fragrance composition according to any one of claims 3 to 7.

11. A hair cosmetic comprising a fragrance composition according to any one of claims 3 to 7.

12. A method of using a cyclopropane compound according to claim 1 or 2 as a fragrance component.

13. A method of producing a cyclopropane compound represented by Formula (I), comprising cyclopropanating a compound represented by Formula (II) using iodomethane to obtain a cyclopropane compound represented by Formula (1): 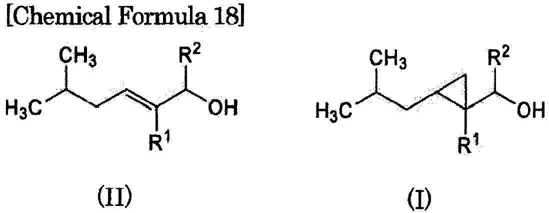 wherein in Formula (I) and Formula (II), R¹ is a methyl group and R² is a methyl group, or R¹ is a hydrogen atom and R² is an ethyl group.

14. Use of a fragrance composition according to any one of claims 3 to 7 as a fragrance component.

15. Use of a cyclopropane compound represented by Formula (I) according to claim 1 or 2 as a fragrance material.

## Patentansprüche

1. Cyclopropanverbindung, dargestellt durch Formel (I): 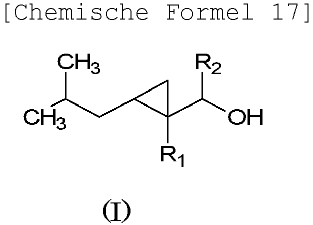 wobei R¹ eine Methylgruppe ist und R² eine Methylgruppe ist, oder R¹ ein Wasserstoffatom ist und R² eine Ethylgruppe ist.

2. Cyclopropanverbindung gemäß Anspruch 1, wobei R¹ eine Methylgruppe ist und R² eine Methylgruppe ist.

3. Duftstoffzusammensetzung, umfassend eine Cyclopropanverbindung gemäß Anspruch 1 oder 2.

4. Duftstoffzusammensetzung, umfassend eine Cyclopropanverbindung gemäß Anspruch 1, in welcher R¹ eine Methylgruppe ist und R² eine Methylgruppe ist, und eine Cyclopropanverbindung gemäß Anspruch 1, in welcher R¹ ein Wasserstoffatom ist und R² eine Ethylgruppe ist.

5. Duftstoffzusammensetzung gemäß Anspruch 4, wobei das Massenverhältnis einer Cyclopropanverbindung gemäß Anspruch 1, in welcher R¹ eine Methylgruppe ist und R² eine Methylgruppe ist, zu einer Cyclopropanverbindung gemäß Anspruch 1, in welcher R¹ ein Wasserstoffatom ist und R² eine Ethylgruppe ist (Cyclopropanverbindung gemäß Anspruch 1, in welcher R¹ eine Methylgruppe ist und R² eine Methylgruppe ist)/(Cyclopropanverbindung gemäß Anspruch 1, in welcher R¹ ein Wasserstoffatom ist und R² eine Ethylgruppe ist) 70/30 bis 99,9/0,1 ist.

6. Duftstoffzusammensetzung gemäß einem der Ansprüche 3 bis 5, ferner umfassend ein oder mehrere andere Duftstoffmaterialien ausgewählt aus der Gruppe bestehend aus Alkoholen, Kohlenwasserstoffen, Phenolen, Estern, Aldehyden, Ketonen, Acetalen und anderen Ethern als der durch Formel (I) dargestellten Cyclopropanverbindung.

7. Duftstoffzusammensetzung gemäß einem der Ansprüche 3 bis 6, ferner umfassend ein Öl.

8. Faserbehandlungszusammensetzung, umfassend eine Duftstoffzusammensetzung gemäß einem der Ansprüche 3 bis 7.

9. Weichmacher, umfassend eine Duftstoffzusammensetzung gemäß einem der Ansprüche 3 bis 7.

10. Reinigungsmittelzusammensetzung, umfassend eine Duftstoffzusammensetzung gemäß einem der Ansprüche 3 bis 7.

11. Haarkosmetik, umfassend eine Duftstoffzusammensetzung gemäß einem der Ansprüche 3 bis 7.

12. Verfahren zur Verwendung einer Cyclopropanverbindung gemäß einem der Ansprüche 1 oder 2 als Duftstoffkomponente.

13. Verfahren zur Herstellung einer Cyclopropanverbindung dargestellt durch Formel (I), umfassend die Cyclopropanierung einer durch Formel (II) dargestellten Verbindung unter Verwendung von Iodmethan, um eine durch Formel (I) dargestellte Cyclopropanverbindung zu erhalten: 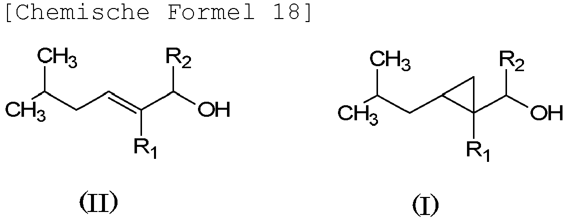 wobei in Formel (I) und Formel (II), R¹ eine Methylgruppe ist und R² eine Methylgruppe ist, oder R¹ ein Wasserstoffatom ist und R² eine Ethylgruppe ist.

14. Verwendung der Duftstoffzusammensetzung gemäß einem der Ansprüche 3 bis 7 als Duftstoffkomponente.

15. Verwendung der durch Formel (I) dargestellten Cyclopropanverbindung gemäß einem der Ansprüche 1 oder 2 als Duftstoffmaterial.

## Revendications

1. Composé cyclopropane représenté par la formule (I) : 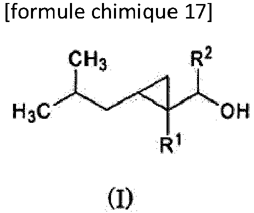 dans lequel R¹ est un groupe méthyle et R² est un groupe méthyle, ou R¹ est un atome d'hydrogène et R² est un groupe éthyle.

2. Composé cyclopropane selon la revendication 1, dans lequel R¹ est un groupe méthyle et R² est un groupe méthyle.

3. Composition de parfum comprenant un composé cyclopropane selon la revendication 1 ou 2.

4. Composition de parfum comprenant un composé cyclopropane selon la revendication 1 dans lequel R¹ est un groupe méthyle et R² est un groupe méthyle, et un composé cyclopropane selon la revendication 1 dans lequel R¹ est un atome d'hydrogène et R² est un groupe éthyle.

5. Composition de parfum selon la revendication 4, dans laquelle le rapport massique entre un composé cyclopropane selon la revendication 1 dans lequel R¹ est un groupe méthyle et R² est un groupe méthyle et un composé cyclopropane selon la revendication 1 dans lequel R¹ est un atome d'hydrogène et R² est un groupe éthyle (le composé cyclopropane selon la revendication 1 dans lequel R¹ est un groupe méthyle et R² est un groupe méthyle)/(le composé cyclopropane selon la revendication 1 dans lequel R¹ est un atome d'hydrogène et R² est un groupe éthyle) est de 70/30 à 99,9/0,1.

6. Composition de parfum selon l'une quelconque des revendications 3 à 5, comprenant en outre une ou plusieurs matières de parfum sélectionnées dans le groupe constitué par des alcools, des hydrocarbures, des phénols, des esters, des aldéhydes, des cétones, des acétals, et des éthers autres que le composé cyclopropane représenté par la formule (I).

7. Composition de parfum selon l'une quelconque des revendications 3 à 6, comprenant en outre une huile.

8. Composition de traitement de fibres comprenant une composition de parfum selon l'une quelconque des revendications 3 à 7.

9. Adoucissant comprenant une composition de parfum selon l'une quelconque des revendications 3 à 7.

10. Composition de nettoyage comprenant une composition de parfum selon l'une quelconque des revendications 3 à 7.

11. Cosmétique capillaire comprenant une composition de parfum selon l'une quelconque des revendications 3 à 7.

12. Procédé d'utilisation d'un composé cyclopropane selon la revendication 1 ou 2 en tant que composant de parfum.

13. Procédé de production d'un composé cyclopropane représenté par la formule (I), comprenant la cyclopropanation d'un composé représenté par la formule (II) à l'aide d'iodométhane pour obtenir un composé cyclopropane représenté par la formule (1) : 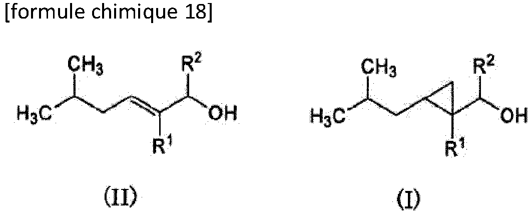 dans lequel dans la formule (I) et la formule (II), R¹ est un groupe méthyle et R² est un groupe méthyle, ou R¹ est un atome d'hydrogène et R² est un groupe éthyle.

14. Utilisation d'une composition de parfum selon l'une quelconque des revendications 3 à 7 en tant que composant de parfum.

15. Utilisation d'un composé cyclopropane représenté par la formule (I) selon la revendication 1 ou 2 en tant que matière de parfum.
